# EUROPÄISCHE PATENTANMELDUNG

(11) **EP 4 814 149 A1**
(43) Veröffentlichungstag der Anmeldung: **30.09.2026**
(21) Anmeldenummer: 25167040.2
(22) Anmeldetag: 28.03.2025
(51) Int. Cl.: C22C 33/04, C21C 5/46, G16C 20/30, G16C 60/00, C21C 5/28, C21C 5/52

(54) **VERFAHREN UND STEUERSYSTEM ZUR HERSTELLUNG VON KALTGEWALZTEN, NIEDRIG- ODER MIKROLEGIERTEN STÄHLEN**

(71) Anmelder: voestalpine Stahl GmbH, 4020 Linz (AT)
(72) Erfinder: Sonnleitner, Markus, 4492 Hofkirchen / Trkr. (AT); Ploberger, Mario, 4020 Linz (AT); Neundlinger, Lukas, 4175 Herzogsdorf (AT)
(74) Vertreter: WSL Patentanwälte Partnerschaft mbB

(57) **Zusammenfassung**

Die Erfindung betrifft ein Verfahren zur Herstellung von kaltgewalzten, niedrig- oder mikrolegierten Stählen eines Zielbereichs der Zugfestigkeit Rₘ, welche durch eine Ziellegierung umfassend Eisen, sowie Begleitelemente und Legierungselemente eingestellt werden, wobei ein Lichtbogenofen und/oder LD-Konverter unter Zugabe von zumindest Stahlschrott beladen wird und der Inhalt des Lichtbogenofens und/oder des LD-Konverters erschmolzen wird, wobei aus einer Schmelze eines letzten primärmetallurgischen Aggregates einer Erzeugungsroute mindestens eine Probe entnommen und durch deren Analyse Gehalte der Begleit- und Legierungselemente bestimmt werden, wobei Analysen-Ist-Werte der Begleit- und Legierungselementgehalte an ein Steuersystem geleitet werden, wobei Begleitelemente Elemente sind, welche in die Schmelze durch Einsatzstoffe eingebracht werden und deren Gehalte einen durch die Ziellegierung definierten Maximalgehalt nicht überschreiten dürfen und Legierungselemente Elemente sind, die gezielt entsprechend durch die Ziellegierung definierter Zielwerte zugesetzt werden, wobei zwischen Einfluss- und Kompensationselementen unterschieden wird, wobei Einflusselemente und Kompensationselemente Elemente sind, welche einen Einfluss bzw. eine Wirkung auf die Zugfestigkeit Rₘ haben, wobei der Unterschied der Analysen-Ist-Werte von Schwellwerten bezüglich der Einflusselemente erfasst wird, wobei die Größe des Einflusses der Abweichung der Analysen-Ist-Werte von den Schwellwerten aller Einflusselemente auf die Zugfestigkeit Rₘ erfasst wird, wobei zumindest ein Kompensationselement ermittelt wird, welches angepasst, d.h. gemäß einem korrigierten Zielwert, derart in die Schmelze zulegiert wird, dass die Zugfestigkeit Rₘ in einen Zielbereich gebracht und der Einfluss des Einflusselements hierdurch kompensiert wird, wobei die Einflusselemente, deren Einfluss auf die Zugfestigkeit Rₘ kompensiert werden soll, eines, mehrere oder alle aus der Gruppe von Cr, Cu, Mo, Ni umfassen und wobei als Kompensationselemente eines, mehrere oder alle aus der Gruppe von C, Si, Mn, Nb, Ti, N gewählt werden, sowie ein Steuersystem zur Durchführung des erfindungsgemäßen Verfahrens.

## Beschreibung

Die Erfindung betrifft ein Verfahren zur Herstellung von kaltgewalzten, niedrig- oder mikrolegierten Stählen. Ferner betrifft die Erfindung ein Steuersystem zur Herstellung von kaltgewalzten, niedrig- oder mikrolegierten Stählen.

Für die primärmetallurgische Herstellung von Stahl wird zunächst Eisen aus Eisenerz gewonnen, indem dieses zusammen mit Koks und Zuschlagstoffen im Hochofen geschmolzen wird. Das geschmolzene Eisen wird anschließend im Konverterverfahren mit Sauerstoff oder im Elektrolichtbogenofen mit Roheisen, Eisenschwamm oder Schrott als Einsatzmaterial (einzeln oder in Kombination) zu Rohstahl weiterverarbeitet.

Anschließend wird der Rohstahl in der Sekundärmetallurgie weiterbehandelt, um die Zusammensetzung und Reinheit zu verbessern. Hierbei wird der Stahl in speziellen Pfannenöfen und/oder Vakuumbehandlungsanlagen behandelt, wo Zusätze wie Legierungselemente hinzugefügt werden, um die gewünschte chemische Zusammensetzung und spezifische Eigenschaften des Stahls zu erzielen.

Im Hochofen wird normalerweise kein Schrott eingesetzt, da der Prozess primär auf der Reduktion von Eisenerz basiert, um flüssiges Roheisen zu erzeugen. Schrott wird allerdings in der Stahlproduktion in anderen Prozessschritten genutzt, vor allem in Elektrolichtbogenöfen oder in Konvertern.

Das Roheisen, das im Hochofen hergestellt wurde, wird anschließend in einem Sauerstoffkonverter weiterverarbeitet. In solchen Konvertern, beispielsweise in einem LD-Konverter (Linz-Donawitz-Verfahren), wird flüssiges Roheisen durch Einblasen von Sauerstoff entkohlt und in Rohstahl umgewandelt. Dabei wird Schrott als Kühlmittel und zusätzliches Material zugegeben. Die Zugabe von Schrott im Konverterprozess hilft, die Temperatur zu kontrollieren, da exotherme Reaktionen mit Sauerstoff die Schmelze stark erhitzen.

Ein anderer wichtiger Produktionsweg in der Stahlindustrie ist der Elektrolichtbogenofen (electric arc furnace, EAF). Hier wird Schrott verwendet, der durch elektrische Energie eingeschmolzen wird, um flüssigen Stahl herzustellen. Dieses Verfahren ist besonders umweltfreundlich, da es hauptsächlich recyceltes Material verwendet und weniger CO₂-Emissionen verursacht als der Hochofenprozess.

Beim Elektrolichtbogenofenverfahren wird Stahl mit elektrischer Energie aufgeschmolzen. Dabei wird ein elektrischer Lichtbogen zwischen Graphitelektroden und dem Schrottmaterial im Ofen erzeugt, wodurch hohe Temperaturen (bis zu 3500 °C im Brennpunkt des Lichtbogens) entstehen, die den Schrott schmelzen und ihn so zu flüssigem Stahl verarbeiten.

Die Verwendung von Schrott als Rohstoff für Stahl bietet mehrere Vorteile. Das Einschmelzen von Schrott benötigt weniger Energie als die Primärproduktion von Stahl aus Eisenerz. Außerdem reduziert Schrottrecycling die CO₂-Emissionen und den Bedarf an neuen Rohstoffen, was die Umweltbelastung senkt. Da Stahl immer wieder recycelt werden kann, ist die Nutzung von Schrott ein wichtiger Beitrag zur Kreislaufwirtschaft in der Stahlindustrie.

Obwohl die Schrottverwendung im Elektrolichtbogenofen (EAF) viele Vorteile mit sich bringt, gibt es auch einige Nachteile und Herausforderungen, die berücksichtigt werden müssen.

Die erste Herausforderung bei der Verwendung von Schrott ist die schwankende Schrottqualität. Der Schrott, der im EAF verwendet wird, ist oft inhomogen und kann Verunreinigungen enthalten, die sich negativ auf die Stahlqualität auswirken. Zum Beispiel können unerwünschte Elemente wie Kupfer (Cu), Chrom (Cr), Nickel (Ni) und Molybdän (Mo) im Schrott vorkommen, die aus dem Stahl schwer zu entfernen sind, seine Anwendungseigenschaften (u.a. Festigkeit) verändern und ihn spröde oder unbrauchbar für bestimmte Anwendungen machen können. Verunreinigungen können den EAF-Prozess komplizierter und teurer machen, da zusätzliche Aufbereitungsschritte oder Sortierungen erforderlich sein können.

Die zweite große Herausforderung betrifft die Kontrolle der chemischen Legierungszusammensetzung. Da Schrott unterschiedliche Legierungen und Metallzusammensetzungen umfassen kann, kann es schwer sein, die chemische Zusammensetzung des Endprodukts präzise zu steuern. Die Zugabe von Legierungselementen in die Schmelze wird daher komplizierter, und es sind oft zusätzliche Anpassungen nötig, um die gewünschte Stahlspezifikation zu erreichen.

Aufgrund der möglichen Verunreinigungen und der schwankenden Qualität des Schrotts ist es schwer, mit dem EAF-Verfahren niedrigste Gehalte an Elemente wie Cu, Cr, Ni und Mo zu gewährleisten, welche für bestimmte spezialisierte Anwendungen (z.B. im Maschinenbau und in der Automobilindustrie) erforderlich sind. In solchen Fällen ist die Nutzung von Primärmaterialien oder sehr hochwertigem, sortenreinem Schrott notwendig, was die Kosten erhöht.

Neben der chemischen Zusammensetzung, welche in einem Elektrolichtbogenofenprozess nur schwer präzise eingestellt werden kann und deshalb oft in vorbestimmten Toleranzgrenzen, definiert durch Minimal- und Maximalgehalte, gehalten wird, sind auch die Auswirkungen einzelner Elemente auf die Eigenschaften des Endprodukts zu berücksichtigen.

Insbesondere bei einer nachfolgenden Wärmebehandlung oder einer mechanischen Bearbeitung, wie beispielsweise Walzen oder Schmieden, werden die mechanischen Eigenschaften des Stahls zusätzlich gesteuert.

Es sind bereits einige Lösungen bekannt, welche die unerwünschten Elemente in der Legierungszusammensetzung berücksichtigen oder zusätzliche Anpassungen der Legierungselemente vorsehen.

Es ist üblich, fest definierte Zielbereiche der Legierungselemente für eine Stahlgüte festzulegen, welche den minimalen und den maximalen Wert je Legierungselement vorsehen. Für Begleitelemente, welche nicht bewusst legiert werden, aber in den Einsatzstoffen in unbekannter Konzentration enthalten sind, existieren Schwellwerte, welche garantieren, dass bei Unterschreitung dieser Gehalte die Werkstoffeigenschaften innerhalb des gewünschten Bereiches bleiben. Auf den tatsächlichen Gehalt dieser Begleitelemente wird in der Flüssigphase allerdings nicht reagiert.

Dabei ist von Nachteil, dass niedrige Schwellwerte der Begleitelemente die Verwendung von Einsatzstoffen mit geringen Gehalten dieser Elemente erzwingen. Dies wird in erster Linie durch einen hohen Anteil an aus Primärstoffen (Eisenerz) erzeugten Einsatzstoffen erreicht.

Zur Senkung der CO₂-Emissionen ist ein möglichst hoher Anteil an sekundären Einsatzstoffen (beispielsweise Schrott) im Einsatzmix von Vorteil. Dadurch vergrößern sich sowohl die Gehalte als auch die Streuung der Begleitelemente und somit auch die Streuung in den Werkstoffeigenschaften.

Aus der EP 3 956 481 B1 ist ein computergestütztes Verfahren zur Überwachung des Stahlherstellungsprozesses in einem Konverter bekannt. Dabei werden verschiedene Materialien mit spezifischen Eigenschaften in den Konverter eingebracht, um flüssigen Stahl und Schlacke zu produzieren. Bei dem Verfahren werden zuerst die gewünschten Eigenschaften des zu produzierenden flüssigen Stahls und der Schlacke festgelegt. Dann werden die erforderlichen Mengen jedes Materials, die in den Konverter eingebracht werden müssen, berechnet, um die definierten Zielmerkmale des Stahls und der Schlacke zu erreichen. Die berechneten Materialmengen werden an den automatisch steuerbaren Bediener oder an automatische Ladesysteme übermittelt und der Konverter wird entsprechend beladen.

Somit wird hier das Beladen des Konverters mit verschiedenen Ausgangsstoffen beschrieben. Nachteilig ist, dass dabei lediglich die Zusammensetzung der im Konverter erzeugten Schmelze kontrolliert wird. Eine nachträgliche Einflussnahme auf die Legierungszusammensetzung wird nicht spezifiziert.

Aus der DE 10 2021 211 320 A1 ist ein Verfahren zur Steuerung und Regelung einer Produktionsanlage für Walzprodukte aus metallischen Legierungen wie Stahl, Eisen oder Aluminium bekannt. Ziel des Verfahrens ist es, den Einsatz von Rohstoffen zu optimieren und die Produktionskosten zu senken. Anfangs werden für jeden Auftrag spezifische Zielwerte für Material-, Oberflächen- und geometrische Eigenschaften des Endprodukts sowie zulässige chemische Zusammensetzungen mit definierten Toleranzbereichen festgelegt. Durch Anwendung von Prozessmodellen werden für jeden Auftrag prädiktive Ist-Werte der Produkteigenschaften berechnet und mit den Zielwerten verglichen. Nur Zusammensetzungen, die innerhalb der vorgegebenen Toleranzen liegen, werden ausgewählt. Für jede ausgewählte chemische Zusammensetzung werden Kostenparameter wie Legierungs-, Schrott-, Energie-, Eisen-, Zuschlagsstoff- und CO₂-Kosten ermittelt. Diese werden in Form von Strafpunkten oder einer Straffunktion quantifiziert. Anschließend wird die Schnittmenge der zulässigen chemischen Zusammensetzungen für verschiedene Kombinationen von Produktionsaufträgen, die in einer gemeinsamen Charge erschmolzen werden sollen, ermittelt.

Nachteilig dabei ist, dass hier Toleranzbereiche für jede Zusammensetzung ermittelt werden. Eine genaue Steuerung der Zusammensetzung ist mit diesem Modell nicht möglich.

Aus der EP 4 183 498 A1 ist ein KI-basiertes Prognosemodell bekannt, mit welchem sich die mechanischen Eigenschaften aus einigen vorher gemessenen Ausganswerten, wie beispielsweise der Zusammensetzung der Schmelze, vorhersagen lassen. Um einen Einfluss auf die mechanischen Eigenschaften zu nehmen, wird als Output eine Anpassung der Herstellungsparameter, wie beispielsweise Vorwalzverhältnis, Fertigwalzverhältnis, Walzstarttemperatur, Startzeit der Kühlung, Abkühlungsrate oder Liniengeschwindigkeit vorgeschlagen.

Nachteilig dabei ist, dass die Kombination eines maschinellen Lernmodells mit einem metallurgischen Modell hohe Rechenleistung und eine umfangreiche Datenerfassung erfordert. Die Modelle müssen kontinuierlich trainiert und aktualisiert werden, was zeit- und ressourcenaufwendig sein kann. Außerdem ist das Modell auf spezifische Produktionslinien und -prozesse optimiert. Wenn die Produktionsbedingungen oder Anforderungen schnell geändert werden müssen, kann das System Schwierigkeiten haben, sich anzupassen.

Aufgabe der Erfindung ist es, ein Verfahren zur Herstellung von kaltgewalzten, niedrig- oder mikrolegierten Stählen zur Verfügung zu stellen, mit welchem konstante Produkteigenschaften auch bei größeren Schwankungen der Begleitelementfracht gewährleistet werden.

Die Aufgabe wird mit einem Verfahren mit den Merkmalen des Anspruchs 1 gelöst.

Vorteilhafte Weiterbildungen sind in den Unteransprüchen gekennzeichnet.

Ferner ist die Aufgabe der Erfindung, ein System zur Herstellung von kaltgewalzten, niedrig- oder mikrolegierten Stählen zur Verfügung zu stellen, mit welchem es gelingt, konstante Produkteigenschaften auch bei größeren Schwankungen der Begleitelementfracht zu gewährleisten.

Die Aufgabe wird mit einem Steuersystem mit den Merkmalen des Anspruchs 14 gelöst.

Es handelt sich hierbei um das Konzept des dynamischen Legierens. Dabei wird eine Adaption der Zielwerte für Legierungselemente in der Flüssigphase durchgeführt. Mit Unterstützung eines Prognosemodells wird der Einfluss der gemessenen Gehalte der Begleitelemente auf die Werkstoffeigenschaften abgebildet, und mittels eines Korrekturmodells wird eine Reaktion in Form von adaptierten Zielwerten von Elementen, welche legiert werden, vorgeschlagen.

Die Erfinder haben erkannt, dass bei der dynamischen Korrektur der Elementgehalte zwischen zwei Elementarten unterschieden werden soll. Es handelt sich hierbei um Einfluss- und Kompensationselemente.

Bei der Stahlherstellung werden beim Herstellungsprozess verschiedene Einsatzstoffe verwendet. Dies sind zum Beispiel Eisenerz, Koks, Schlackenbildner, Eisenschwamm, Schrott.

Über diese Einsatzstoffe wird natürlich nicht nur reines Eisen eingebracht, sondern auch sogenannte Begleitelemente und Spurenelemente.

Begleitelemente sind üblicherweise nicht erwünschte Elemente und werden im Verlauf eines nachfolgenden metallurgischen Prozesses entfernt, sofern das möglich ist. Ist ein Entfernen nicht möglich, müssen das jeweilige Begleitelement und sein Einfluss bei metallurgischen Prozessen berücksichtigt werden.

Für jede Legierung, welche durch die chemische Zusammensetzung definiert wird, existiert eine definierte Begleitelementklasse, d.h. es sollen bestimmte Schwellwerte der Begleitelemente nicht überschritten werden.

Die Legierung ist eine Grundvoraussetzung für das Erreichen der zu erzielenden oder geforderten Eigenschaften des Endprodukts.

Bei Spurenelementen handelt es sich um explizit störende Begleitelemente (z.B. Arsen), welche in sehr geringen Mengen vorliegen. Diese werden im Zuge des (hier durchgeführten dynamischen) Legierens nicht berücksichtigt.

Legierungselemente sind Elemente, welche dem Stahl gezielt zugesetzt (zulegiert) werden, um dessen Werkstoffeigenschaften auf die gewünschte Weise zu verbessern. Solche Werkstoffeigenschaften können mechanische Eigenschaften wie Härte, Zugfestigkeit, Zähigkeit und weitere Eigenschaften sein, aber auch chemische Eigenschaften wie Widerstand gegen Korrosion oder Wasserstoffversprödung und weitere.

Die Legierungselemente müssen Teil der Schmelze sein, um eine gewünschte Stahlsorte mit definierter Ziellegierungszusammensetzung herzustellen. Letztere gewährt gewisse Toleranzen bezüglich der einzelnen Legierungselementgehalte, d.h. innerhalb der Stahlsorte sind für die Legierungselemente ein Minimal- und ein Maximalgehalt vorgesehen.

Bei Schrotteinsatz muss beachtet werden, dass Schrott für sich gesehen Begleitelemente und Spurenelemente aus seiner Rohstoffhistorie und Legierungselemente aus seiner metallurgischen Historie enthält.

Wird nun der Schrott als Einsatzstoff in der Stahlherstellung verwendet, sind seine Bestandteile, bis auf Eisen, für das Produkt, was unter Einsatz des Schrotts erzeugt werden soll, Begleitelemente, Spurenelemente oder Legierungselemente.

Deren Anwesenheit muss also berücksichtigt werden, falls sie (als ehemalige Legierungselemente) aus dem Produkt über metallurgische Prozesse nicht ohne weiteres oder wirtschaftlich entfernbar sind. Jedoch kann ihre Wirkung erfindungsgemäß berücksichtigt und kompensiert werden.

Im klassischen Herstellungsprozess wird von einem Produkt (Stahl) ausgegangen, welches entsprechend den Marktanforderungen bestimmte werkstoffkundliche und insbesondere mechanische und/oder chemische Eigenschaften aufweist. Auf dieses Anforderungsprofil hin, wird es chemisch/metallurgisch eingestellt. Ein solches Produkt ist also eine Legierung, wobei die Legierungsbestandteile durch einen definierten Minimalgehalt und einen Maximalgehalt bestimmt sind. Von einer bestimmten Legierung ist somit bekannt, welche Eigenschaften sie haben wird.

Wird nun aufgrund geänderter Bedingungen, der Schrotteinsatz erheblich erhöht, wird das Produkt folgerichtig mit einer höheren Menge von ehemaligen Legierungselementen, Begleitelementen und Spurenelementen befrachtet. Diese Elemente haben einen Einfluss zum Beispiel auf mechanische Eigenschaften des Produkts.

Hier setzt die Erfindung dadurch an, dass zu den Spurenelementen, Begleitelementen und ehemaligen Legierungselementen, die einen Einfluss auf eine oder mehrere gewünschte Eigenschaften haben (nachfolgend Einflusselemente genannt), Legierungselemente ausgewählt werden, die den Einfluss der Einflusselemente auf einen gewünschten Einflussumfang kompensieren können (nachfolgend Kompensationselemente genannt).

Einflusselemente sind Spurenelemente, Begleitelemente und ehemalige Legierungselemente, welche aus Einsatzstoffen eingebracht werden und deren Gehalt in der Schmelze einen definierten Schwellwert überschreiten kann.

Kompensationselemente sind Elemente, welche im besten Fall reguläre, d.h. in der Ziellegierung vorgesehene Legierungselemente sind und deren durch Einsatzstoffe eingebrachter Gehalt unter einem durch die Ziellegierung definierten Zielwert liegt. Dies schließt nicht aus, dass in bestimmten Fällen ein Element als Kompensationselement ausgewählt wird, welches kein reguläres Legierungselement ist. Als Beispiel könnte der unerwünschte Einfluss der sogenannten Rotbrüchigkeit durch Kupfer durch die Zugabe von Nickel kompensiert werden. Ist Nickel kein reguläres Legierungselement der Ziellegierung, könnte es dennoch als Kompensationselement Anwendung finden.

Sollte ein zur Kompensation des Einflusses eines Einflusselements auf eine erste (mechanische) Eigenschaft ausgewähltes erstes Kompensationselement zusätzlich einen Einfluss auf eine zweite (mechanische) Eigenschaft haben, kann es notwendig werden, diesen Einfluss durch ein zweites Kompensationselement zu kompensieren. Das erste Kompensationselement wäre in diesem Fall zugleich ein Einflusselement.

In bestimmten Grenzfällen kann es sogar notwendig werden, die durch die Ziellegierung definierten Zielwerte bestimmter Legierungselemente (nach oben) anzupassen, wenn zur Kompensation eine den Maximalgehalt übersteigende Menge zugegeben werden muss.

Ein einfaches Beispiel soll dies deutlich machen. Neben Eisen sind in einer gewünschten Stahllegierung die Elemente A, B und C enthalten. A ist ein Einflusselement und nun durch die Einsatzstoffe in einer Menge vorhanden, die den Schwellwert überschreitet. Wenn das Kompensationselement C dann einen vergleichbaren Einfluss auf eine gewünschte Eigenschaft hat wie A, jedoch in der Legierung wenig oder gar nicht vorhanden ist, wird hiervon nur so viel zugesetzt, dass die addierte Wirkung von A und C gemeinsam die gewünschte Eigenschaft in einen Zielbereich bringt. D.h. in diesem Fall, dass vom ursprünglichen Zielwert des Kompensationselements C abgewichen wird, indem weniger C zugesetzt wird (adaptierter Zielwert), um den zu hohen A-Gehalt zu kompensieren.

Ein weiteres Beispiel ist, dass, wenn das Einflusselement A in einer Menge vorhanden ist, die den Schwellwert übersteigt, und C ein Kompensationselement ist, welches den Einfluss von A auf eine gewünschte Eigenschaft verringert, C in einer Menge zulegiert wird, welche zusätzlich zur eigenen Wirkung noch die Wirkung von A kompensiert.

Kompensation im Sinne der Erfindung bedeutet somit, dass eine Anpassung der Legierungszusammensetzung nach oben oder unten stattfinden kann, um den Einfluss auf mechanische Eigenschaften bzw. Abweichungen der mechanischen Eigenschaft durch Einflusselemente zu kompensieren.

B ist in diesem Fall z.B. ein Element, welches auf die gewünschte Eigenschaft, beispielsweise Festigkeit, keinen Einfluss hat.

Insgesamt kann durch dieses Vorgehen die Schrottvorauswahl gröber erfolgen, wodurch sich die Effizienz des Gesamtprozesses bezüglich Kosten, Zeit und Ressourcen erhöht.

Eingangskenngrößen in das Prognosemodell sind Einflusselemente, deren Gehalte in der Flüssigphase nicht mehr geändert werden, wie beispielsweise Cr, Cu, Mo, Ni.

Ausgangsgrößen des Korrekturmodells sind adaptierte Zielwerte von Elementen, welche in der Flüssigphase legiert werden. Es handelt sich hierbei um die sogenannten Kompensationselemente, wie beispielsweise C, Si, Mn, Nb, Ti, N. Kompensationselemente sind somit Elemente, welche in der Flüssigphase zulegiert werden, um den Einfluss der Einflusselemente auszugleichen, damit die Ziel-Eigenschaften der Ziellegierung (definiert durch die gewünschte Stahlsorte) erreicht werden.

Wie oben schon ausgeführt, können einige Elemente auch dann Kompensationselemente sein, wenn sie keine Legierungselemente der Ziellegierung sind. Da diese als Kompensationselemente eingesetzten Elemente in der Ziellegierung auch einen (weiteren) Einfluss entfalten können, sind sie diesbezüglich auch Einflusselemente, deren Einfluss ggf. wiederum mit einem (weiteren) Kompensationselement kompensiert werden muss.

Das Prognosemodell bestimmt die Wirkung bzw. den Einfluss der Abweichungen der Einflusselemente, die ihre Schwellwerte überschreiten, von ihren jeweiligen Schwellwerten auf die Zugfestigkeit Rₘ. Eingangskenngrößen dafür sind die Abweichungen der Einflusselementgehalte von den Schwellwerten.

Eine einfache Implementation des Korrekturmodells nutzt die tatsächlichen (auf Probenanalysen beruhenden) Abweichungen der Gehalte der Einflusselemente von ihren jeweiligen Schwellwerten sowie Variationen von Abweichungen der Gehalte der Kompensationselemente von ihren jeweiligen Zielwerten als Eingangsgrößen.

In der Stahlherstellung wird "Schrott" als recycelter, wiederverwendbarer metallischer Sekundärstoff bezeichnet, der als Rohmaterial zur Produktion von neuem Stahl dient. Schrott besteht hauptsächlich aus Eisen und Stahl und wird in verschiedenen Qualitäten und Sorten klassifiziert, je nach Reinheit, Form, Größe und chemischer Zusammensetzung. Schrottsorten sind dabei: Altschrott, Neuschrott, Schredderschrott usw.

Das vorliegende Verfahren, bei welchem Schrott eingesetzt wird, betrifft die Herstellung kaltgewalzten, niedrig- oder mikrolegierten Stahls.

Kaltgewalzte hochfeste, niedrig- oder mikrolegierte Stähle (High Strength Low Alloyed - HSLA) sind Teil der Produktfamilie der konventionell höherfesten Stähle. Sie bieten eine Vielfalt an Streckgrenzenvarianten und decken den oberen Festigkeitsbereich der konventionell höherfesten Stähle ab. Sie zeichnen sich durch ein hohes Streckgrenzenverhältnis, eine gute Kaltumformbarkeit und gute Schweißeignung aus. Die einzelnen Festigkeitsklassen werden im Wesentlichen durch die Zugabe von Mikrolegierungselementen, wie Niob, Titan und/oder Vanadium, eingestellt. Diese Legierungselemente können einzeln oder in Kombination zugesetzt werden und bewirken durch Kornfeinung und Ausscheidungshärtung eine Festigkeitssteigerung. Zusätzlich werden Kohlenstoff und mischkristallverfestigende Elemente zur Festigkeitssteigerung verwendet. Mikrolegierte Stähle bieten durch ihre Vielfalt an Streckgrenzenvarianten die Möglichkeit eines optimal bauteilbezogenen Werkstoffeinsatzes und eignen sich somit besonders für die Fertigung von Struktur-, Chassis- und Verstärkungsteilen.

Allgemein bieten solche kaltgewalzten, niedrig- oder mikrolegierten Stähle folgende Vorteile:
- Hohe Vielfalt an Festigkeitsvarianten mit Streckgrenzen bis 670 MPa (längs zur Walzrichtung);
- Hohes Streckgrenzenverhältnis;
- Sehr gute Kaltumformbarkeit;
- Gute Schweißeignung
- Korrosionsbeständigkeit durch Beschichtungen auf Zinkbasis, zum Beispiel ZE-, Z-, ZF- bzw. EG-, GI-, GA- oder ZM-Beschichtungen.

Das Konzept beruht darauf, kaltgewalzten, niedrig- oder mikrolegierten Stahl einer vorgegebenen Güte zu erzeugen, bei dem sich insbesondere die Zugfestigkeit Rₘ trotz erhöhter Einflusselementgehalte, welche durch den Schrott im Elektrolichtbogenofen eingebracht werden, gar nicht oder nur möglichst geringfügig ändert. Insbesondere wird die Änderung der Zugfestigkeit im Vergleich zur über die Primärroute ohne oder mit geringem Schrotteinsatz erreichten Zugfestigkeit betrachtet (z.B. beim LD-Verfahren).

Dies geschieht durch Abgabe einer Prognose hinsichtlich der Wirkung der Einflusselemente auf die Zugfestigkeit Rₘ mittels eines Prognosemodells und anschließende Adaption der Zielwerte eines oder mehrerer Kompensationselemente durch Anwendung eines Korrekturmodells. Dieses Konzept wird nachfolgend als dynamisches Legieren bezeichnet.

Im vorliegenden Verfahren wird die Wirkung der Elemente als Steigung bzw. Faktor der Festigkeitsänderung mit variierendem Legierungsgehalt bezeichnet.

Ausgehend von einer durch die vorgegebene Stahlsorte definierten Ziellegierungszusammensetzung, werden die Abweichungen der Einflusselemente von ihren jeweiligen Schwellwerten bestimmt. Mittels eines Prognosemodells wird dann die Wirkung dieser Abweichungen hinsichtlich der Einflusselementgehalte auf die Zugfestigkeit Rₘ berechnet. Anschließend wird mithilfe eines Korrekturmodells die notwendige Reduktion bzw. Adaption eines oder mehrerer Kompensationselemente berechnet (adaptierte Zielwerte). Das Korrekturmodell kann dabei auch weitere Restriktionen oder spezifische Kundenanforderungen berücksichtigen.

Demnach umfasst das Verfahren für das dynamische Legieren zwei zusammenwirkende Komponenten. Die erste Komponente betrifft ein Prognosemodell, und die zweite Komponente ist ein Korrekturmodell.

Die Analysen-Ist-Werte der in der Schmelze enthaltenen Elemente können laufend zunächst im letzten primämetallurgischen Aggregat, im Konverter, im Elektrolichtbogenofen, in der Pfanne oder dazwischen durch Analyse ermittelt werden. Die Analysen-Ist-Werte können vor einem jeglichen ersten Legierungsschritt analysiert werden. Basierend auf der Analyse wird eine variable Legierungszugabe, welche sich an den Minimalgehalten der Legierungselemente orientiert, berechnet und nach dem Abstich zulegiert. Anschließend erfolgt eine Spülgasbehandlung zum Homogenisieren der Schmelze und ggf. eine zweite Analyse wird durchgeführt. Basierend auf den ermittelten Analysen-Ist-Werten beginnt das dynamische Legieren.

Anfangs werden die tatsächlichen Werte bewusst unter den Zielwerten der Legierungselemente gehalten. Dies ist nötig, um die weitere Zugabe von Legierungselementen zu ermöglichen.

Mit den Analysen-Ist-Werten der Einflusselemente werden die Abweichungen zu den Schwellwerten bestimmt. Diese Abweichungen resultieren aus der ungewollten Überschreitung der Schwellwerte aufgrund der Verwendung von Einsatzstoffen mit erhöhter Einflusselementfracht. Anschließend wird im Prognosemodell ein Einflussfaktor F_{E} aus den Abweichungen der Einflusselementgehalten von den Schwellwerten berechnet, welcher ein Maß für den Einfluss der Einflusselemente auf die Zugfestigkeit Rₘ darstellt.

Der Einflussfaktor F_{E} in MPa wird im Prognosemodell anhand der folgenden Formel berechnet: ${F}_{E} = 109 \times Δ \left[at%\right] Cr + 64 \times Δ \left[at%\right] Cu + 259 \times Δ \left[at%\right] Mo + 45 \times Δ \left[at%\right] Ni ,$ wobei Δ[at%]X der den Schwellwert übersteigende Gehalt des Einflusselements X ist und wobei die für das jeweilige Einflusselement X ermittelte Steigung, welche dessen Einfluss auf die Zugfestigkeit Rₘ abbildet, um ±25% von dem angegebenen Wert abweichen kann.

Die nötige dynamische Korrektur wird dann mithilfe des Korrekturmodells ermittelt.

Im Korrekturmodell werden dynamisch korrigierte Zielwerte der Kompensationselemente für die gewünschte Legierung auf Basis einer Optimierungsrechnung berechnet, wobei diese um maximal 30%, bevorzugt 25%, besonders bevorzugt 20% gegenüber ihren eigentlichen Zielwerten abgesenkt werden dürfen. Anschließend wird ein Kompensationsfaktor F_{K} aus den Abweichungen der Kompensationselementgehalte von den Zielwerten berechnet. Diese Abweichungen entstehen durch eine gewollt geänderte chemische Zusammensetzung bezüglich der Kompensationselemente.

Der Kompensationsfaktor F_{K} in MPa wird im Korrekturmodell anhand der folgenden Formel berechnet: FK = 207 × Δ[at%]C + 25 × Δ[at%]Si + 96 × Δ[at%]Mn + (3913 - 34544 × [at%]NbZiel) × Δ[at%]Nb2 + 1204 × Δ[at%]Ti - 277 × Δ[at%]N, wobei [at%]Y_{Ziel} der Zielwert des Kompensationselements Y und Δ[at%]Y die Abweichung des im Korrekturmodell zum Ausgleich der Zugfestigkeit Rₘ berechneten Kompensationselementgehalts vom Zielwert des Kompensationselements Y ist und wobei die für das jeweilige Kompensationselement Y ermittelte Steigung, welche dessen Einfluss auf die Zugfestigkeit Rₘ abbildet, um ±25% von dem angegebenen Wert abweichen kann.

Schließlich wird die Summe aus dem Einflussfaktor F_{E} und dem Kompensationsfaktor F_{K} folgendermaßen bestimmt: $Δ F = \left|{F}_{E}+{F}_{K}\right| .$ ΔF soll dabei minimiert werden, sodass ΔF einen Maximalwert, insbesondere 20 MPa, bevorzugt 10 MPa, besonders bevorzugt 5 MPa nicht überschreitet.

Die Berechnung adaptierter Kompensationselementgehalte bzw. Zielwerte und des Kompensationsfaktors F_{K} wird im Korrekturmodell iteriert, bis eine Lösung gefunden wird, welche obige Bedingung für ΔF erfüllt.

Bei der Auswahl der Kompensationselemente können folgende Kriterien herangezogen werden: Möglichkeit der Elementkorrektur innerhalb des Zielbereichs, Treffsicherheit des jeweiligen Kompensationselements, Zeitpunkt der Legierungseinstellung, Stärke der Abhängigkeit der Zugfestigkeit Rₘ von dem jeweiligen Kompensationselement, Differenz aus dem zum Ausgleich der Zugfestigkeit Rₘ berechneten Kompensationselementgehalt und dem Zielwert des Kompensationselements, Wahl weiterer Kompensationselemente, Kosten des jeweiligen Kompensationselements.

Unter der Möglichkeit der Elementkorrektur innerhalb des Zielbereichs ist zu verstehen, dass ein Kompensationselement ausgewählt wird, dessen durch das Korrekturmodell neu berechneter, adaptierter Gehalt nicht vom ursprünglichen, durch die gewünschte Stahlgüte definierten Zielbereich abweicht. Dies ist als besonders vorteilhaft zu sehen.

Unter der Treffsicherheit des jeweiligen Kompensationselements ist zu verstehen, dass ein Kompensationselement ausgewählt wird, dessen Gehalt mit einer hohen Treffsicherheit angepasst werden kann, d.h. es ist bei der Umsetzung des Zulegierens im Rahmen des dynamischen Legierens mit einer nur geringen Abweichung von ±5% von dem angestrebten Gehalt zu rechnen. Solche Kompensationselemente sind bevorzugt auszuwählen.

Der Zeitpunkt der Legierungseinstellung kann ebenfalls ein Auswahlkriterium darstellen. Es kann zum Beispiel vorteilhaft sein zuerst die Kompensationselemente C und Mn einzusetzen und zu einem späteren Zeitpunkt Nb und Ti.

Die Stärke der Abhängigkeit der Zugfestigkeit Rₘ von dem jeweiligen Kompensationselement mag variieren. Es ist vorteilhaft ein Kompensationselement auszuwählen, welches eine starke Wirkung hinsichtlich der Kompensation des Einflusses des Einflusselements auf die Zugfestigkeit Rₘ hat. In diesem Fall kann es ausreichend sein, lediglich eines oder nur wenige Kompensationselemente für das dynamische Legieren heranzuziehen. In diesem Sinne ist zum Beispiel folgende Reihung zu wählen: 1) Nb, 2) Mn, 3) C.

Die Differenz aus dem zum Ausgleich der Zugfestigkeit Rₘ berechneten Kompensationselementgehalt (adaptierter Zielwert) und dem Zielwert des Kompensationselements sollte möglichst klein ausfallen, um erhöhten Ressourcenverbrauch und mögliche Sekundärwirkungen, verursacht durch den geänderten Kompensationselementgehalt, zu vermeiden. Aufgrund ungewollter Sekundärwirkungen könnte nämlich wiederum eine Kompensation des Einflusses des betreffenden Kompensationselements notwendig werden.

Die Wahl weiterer Kompensationselemente kann notwendig werden, sofern ein Kompensationselement nicht in dem Maß angepasst werden kann, dass es die Wirkung der Einflusselemente bereits ausreichend kompensiert. Ist die Wirkung eines Kompensationselements also nicht ausreichend, wird zunächst ein weiteres, zweites Kompensationselement herangezogen. Sind diese in Kombination immer noch nicht ausreichend, um die Wirkung der Einflusselemente zu kompensieren, wird ein weiteres, drittes Kompensationselement herangezogen usw.

Auch die Kosten des jeweiligen Kompensationselements können bei der Auswahl bzw. Reihung der Kompensationselemente berücksichtigt werden. So ist es wirtschaftlich sinnvoll, zunächst kostenintensivere Kompensationselemente einzusetzen, da deren Gehalte im Zuge des dynamischen Legierens in der Regel nach unten korrigiert werden. In diesem Sinne würde die Reihung der Kompensationselemente beispielsweise folgendermaßen ausfallen: 1) Nb, 2) Mn, 3) Si, 4) Ti, 5) C.

Alle diese Kriterien können einzeln oder in Kombination bei der Auswahl der Kompensationselemente eine Rolle spielen.

Der Einfluss der einzelnen Elemente ist wie folgt.

Kupfer wirkt festigkeitssteigernd, vermindert aber zugleich die Warmumformbarkeit und Bruchdehnung. Ebenfalls können Kupferanreicherungen an der Oberfläche beobachtet werden. Bei geringen Gehalten ist die festigkeitssteigernde Wirkung des Kupfers auf Mischkristallverfestigung zurückzuführen. Die geringe Löslichkeit im Stahl kann aber zu niedrigschmelzende Phasen an den Korngrenzen führen und somit die Warmumformbarkeit wesentlich verschlechtern. Dies kann in weiterer Folge zu Rissen führen. Es ist vorteilhaft, wenn der Kupfergehalt 0,17 at% nicht übersteigt. Der Schwellwert von Kupfer, ab dem ein Eingriff durch dynamisches Legieren durchgeführt wird, liegt bei 0,02 at%.

Nickel erhöht durch Mischkristallverfestigung die Festigkeit und kann sich zähigkeitsverbessernd auswirken. Darüber hinaus erhöht Nickel die Löslichkeit von Kupfer. Es ist vorteilhaft, wenn der Nickelgehalt 0,28 at% nicht übersteigt. Der Schwellwert von Nickel, ab dem ein Eingriff durch dynamisches Legieren durchgeführt wird, liegt bei 0,03 at%.

Molybdän bewirkt eine Verzögerung der Austenit-Ferrit-Umwandlung sowie der Rekristallisation. Als Folge davon steigt mit höherem Molybdängehalt die Festigkeit des Stahls. Es ist vorteilhaft, wenn der Molybdängehalt 0,05 at% nicht übersteigt. Der Schwellwert von Molybdän, ab dem ein Eingriff durch dynamisches Legieren durchgeführt wird, liegt bei 0,01 at%.

Chrom erhöht die Festigkeit durch Mischkristallhärtung, kann aber auch durch Verzögerung der Austenitumwandlung solche Gefügebestandteile begünstigen, welche die Stahlfestigkeit zusätzlich erhöhen. Es ist vorteilhaft, wenn der Chromgehalt 0,26 at% nicht übersteigt. Der Schwellwert von Chrom, ab dem ein Eingriff durch dynamisches Legieren durchgeführt wird, liegt bei 0,04 at%.

Kohlenstoff wirkt als interstitielles Atom, gebunden in Zementit oder als Perlit umgewandelt und in Karbiden, wie zum Beispiel NbC und TiC, festigkeitssteigernd. Es ist vorteilhaft, wenn ein Kohlenstoffgehalt von 0,09 bis 0,60 at% gewählt wird.

Silizium wirkt als Mischkristallhärter und steigert die Festigkeit. Es ist vorteilhaft, wenn ein Siliziumgehalt von ≤1,20 at% gewählt wird.

Mangan ist ein mischkristallverfestigendes Element. Es bindet Schwefel zu Mangansulfiden und ist ein starker Austenitstabilisator. Daher senkt es die Ac3-Temperatur. Zu viel Mangan kann den Austenit stabilisieren und verhindert, dass sich Austenit beim Abkühlen in Ferrit und Perlit umwandelt. Mangan neigt zu Seigerungen. Es ist vorteilhaft, wenn ein Mangangehalt von 0,20 bis 1,80 at% gewählt wird.

Nb wird wegen seines Einflusses auf die Korngröße zur Erhöhung der Festigkeit und Zähigkeit verwendet. Nb erhöht die Festigkeit des Stahls durch die Ausscheidung von NbC und/oder Nb(C, N) in der Ferritmatrix. Des Weiteren kann die Rekristallisation und die Phasenumwandlung gehemmt werden, wodurch die Korngröße sinkt und Festigkeit und Zähigkeit des Stahls steigen. Es ist vorteilhaft, wenn der Niobgehalt ≤ 0,06 at% gewählt wird. Titan ist ein starkes Desoxidationsmittel und bindet Stickstoff. Titannitride in Austenit sind bis zum Schmelzpunkt und teilweise darüber stabil. Daher ist Titan in Verbindung mit Stickstoff ein ideales Element, um das Kornwachstum des Austenits unter hohen Temperaturen herabzusetzen. Titancarbide und Titancarbonitride scheiden sich bei niedrigeren Temperaturen aus und verbessern die Festigkeit und Zähigkeit aufgrund des Effektes der Ausscheidungshärtung und seines Einflusses auf eine geringere Korngröße. Es ist vorteilhaft, wenn der Titangehalt ≤ 0,18 at% gewählt wird.

Es vorteilhaft, wenn die Summe Ti + Nb in der Legierung 0,003 bis 0,14 at% beträgt.

Stickstoff bildet in Anwesenheit von Titan und Aluminium korrespondierende Nitride, Titannitride (TiN) und Aluminiumnitride (AlN), die eine übermäßige Vergröberung des Austenitkorns während der Wärmebehandlung des Stahls unterdrücken. Stickstoff wirkt ferner ähnlich wie Kohlenstoff als Austenitbildner. Zur Vermeidung einer Alterung wird N in vielen Stählen durch Zugabe von Legierungselementen abgebunden, z.B. zu AlN, TiN, BN. Es ist vorteilhaft, wenn ein Stickstoffgehalt ≤0,08 at% gewählt wird.

Die Wirkung von Phosphor und Schwefel ist besonders ungünstig, da diese Elemente zu Seigerungen an den Korngrenzen führen, welche die Gefahr von Korngrenzenrissen erhöhen. Zudem erhöhen Phosphor und Schwefel das Risiko für Heißrisse. Die Gehalte von Phosphor und Schwefel müssen daher sehr niedrig gehalten werden (P: ≤ 0,055 at%; S: ≤ 0,045 at%).

Aluminium ist ein optionales Legierungselement, welches die Ac3-Temperatur erhöht, die Ferritbildung fördert und die Ausscheidungshärtung erleichtern kann. In mikrolegierten Stählen ist die Zugabe von Aluminium als Legierungselement nicht zwingend notwendig. Aluminium wird jedoch üblicherweise als Desoxidationsmittel für den Stahl verwendet und ist daher in geringen Mengen vorhanden. Durch Aluminium kann es zur Bildung von Aluminiumnitriden kommen, welche die Korngrenzenbewegung behindern und so zu einer Kornfeinung führen kann. Es ist vorteilhaft, wenn ein Aluminiumgehalt von 0,03 bis 0,40 at% gewählt wird.

Es ist ferner vorteilhaft, wenn die Zugfestigkeit Rₘ zwischen 310 MPa und 780 MPa liegt.

In einer beispielhaften Ausführungsform kann die Zugfestigkeit Rₘ in MPa mit der folgenden Formel berechnet werden: Rm = 235 + 207 × [at%]C + 25 × [at%]Si + 96 × [at%]Mn + 243 × [at%]P - 104 × [at%]S + 18 × [at%]Al - 277 × [at%]N + 3913 × [at%]Nb - 17272 × [at%]Nb2 + 1204 × [at%]Ti + 109 × [at%]Cr + 64 × [at%]Cu + 259 × [at%]Mo + 45 × [at%]Ni, wobei [at%]i der Gehalt des jeweiligen Elements i ist und wobei die für das jeweilige Element i ermittelte Steigung (der Faktor vor dem Elementgehalt), welche dessen Einfluss auf die Zugfestigkeit Rₘ abbildet, um ±25% von dem angegebenen Wert abweichen kann.

Die Steigungen (d.h. Einflüsse der Elemente auf die Zugfestigkeit Rₘ) ergeben sich aus praktischen Versuchen, beispielsweise Laborversuchen, sowie aus der statistischen Auswertung von Prozess- und Versuchsdaten der Serienproduktion.

Weiterhin weist der kaltgewalzte, niedrig- oder mikrolegiertem Stahl in einer beispielhaften Ausführungsform eine Dicke von 0,30 bis 3,0 mm auf und kann unbeschichtet oder mit einer Korrosionsschutzbeschichtung auf Basis von Zink ausgeführt sein.

Es ist zu beachten, dass die schädliche Wirkung von Begleitelementen vor allem in Kombination untereinander als kritisch zu sehen ist, wobei deren Ursprung in entsprechenden Schrottverunreinigung (beim Einsatzmix zur Erstellung der Schmelze) begründet ist. Bei Verwendung von Schmelztechnologien mit erhöhten Schrottmengen können somit durch die unweigerlich erhöhte Begleitelementfracht größere Schwankungsbreiten bezüglich der Performance relevanten Werkstoffeigenschaften auftreten.

Die Erfindung betrifft somit ein Verfahren zur Herstellung von kaltgewalzten, niedrig- oder mikrolegierten Stählen eines Zielbereichs der Zugfestigkeit Rₘ, welche durch eine Ziellegierung umfassend Eisen, sowie Begleitelemente und Legierungselemente eingestellt werden, wobei ein Lichtbogenofen und/oder LD-Konverter unter Zugabe von zumindest Stahlschrott beladen wird und der Inhalt des Lichtbogenofens und/oder des LD-Konverters erschmolzen wird, wobei aus einer Schmelze eines letzten primärmetallurgischen Aggregates einer Erzeugungsroute mindestens eine Probe entnommen und durch deren Analyse Gehalte der Begleit- und Legierungselemente bestimmt werden, wobei Analysen-Ist-Werte der Begleit- und Legierungselementgehalte an ein Steuersystem geleitet werden, wobei Begleitelemente Elemente sind, welche in die Schmelze durch Einsatzstoffe eingebracht werden und deren Gehalte einen durch die Ziellegierung definierten Maximalgehalt nicht überschreiten dürfen und Legierungselemente Elemente sind, die gezielt entsprechend durch die Ziellegierung definierter Zielwerte zugesetzt werden, wobei zwischen Einfluss- und Kompensationselementen unterschieden wird, wobei Einflusselemente und Kompensationselemente Elemente sind, welche einen Einfluss bzw. eine Wirkung auf die Zugfestigkeit Rₘ haben, wobei der Unterschied der Analysen-Ist-Werte von Schwellwerten bezüglich der Einflusselemente erfasst wird, wobei die Größe des Einflusses der Abweichung der Analysen-Ist-Werte von den Schwellwerten aller Einflusselemente auf die Zugfestigkeit Rₘ erfasst wird, wobei zumindest ein Kompensationselement ermittelt wird, welches angepasst, d.h. gemäß einem korrigierten Zielwert, derart in die Schmelze zulegiert wird, dass die Zugfestigkeit Rₘ in einen Zielbereich gebracht und der Einfluss des Einflusselements hierdurch kompensiert wird, wobei die Einflusselemente, deren Einfluss auf die Zugfestigkeit Rₘ kompensiert werden soll, eines, mehrere oder alle aus der Gruppe von Cr, Cu, Mo, Ni umfassen und wobei als Kompensationselemente eines, mehrere oder alle aus der Gruppe von C, Si, Mn, Nb, Ti, N gewählt werden.

Eine vorteilhafte Weiterbildung sieht vor, dass die Einflusselemente, deren Einfluss auf die Zugfestigkeit Rₘ kompensiert werden soll, Cr, Cu, Mo und/oder Ni umfassen.

Eine vorteilhafte Weiterbildung sieht vor, dass als Kompensationselemente C, Si, Mn, Nb, Ti und/oder N gewählt werden.

Eine vorteilhafte Weiterbildung sieht vor, dass im Steuersystem zwei Komponenten verwendet werden: ein Prognosemodell und ein Korrekturmodell, wobei mit dem Prognosemodell ein Einflussfaktor F_{E} ermittelt wird, welcher die Größe des Einflusses der Abweichung der Analysen-Ist-Werte von den Schwellwerten aller Einflusselemente auf die Zugfestigkeit Rₘ beschreibt, und mit dem Korrekturmodell zum Ausgleich der Zugfestigkeit Rₘ erforderliche Kompensationselementgehalte berechnet werden sowie ein Kompensationsfaktor F_{K} ermittelt wird, welcher die Größe eines Einflusses einer Abweichung der berechneten Kompensationselementgehalte von den Zielwerten aller Kompensationselemente auf die Zugfestigkeit Rₘ beschreibt.

Eine vorteilhafte Weiterbildung sieht vor, dass zur Berechnung der Zugfestigkeit Rₘ in MPa die folgende Formel verwendet wird: Rm = 235 + 207 × [at%]C + 25 × [at%]Si + 96 × [at%]Mn + 243 × [at%]P - 104 × [at%]S + 18 × [at%]Al - 277 × [at%]N + 3913 × [at%]Nb - 17272 × [at%]Nb2 + 1204 × [at%]Ti + 109 × [at%]Cr + 64 × [at%]Cu + 259 × [at%]Mo + 45 × [at%]Ni, wobei [at%]i der Gehalt des jeweiligen Elements i ist und wobei die für das jeweilige Element i ermittelte Steigung, welche dessen Einfluss auf die Zugfestigkeit Rₘ abbildet, um ±25% von dem angegebenen Wert abweichen kann.

Eine vorteilhafte Weiterbildung sieht vor, dass im Prognosemodell der Einflussfaktor F_{E} in MPa für die Einflusselemente unter Verwendung der folgenden Formel berechnet wird: ${F}_{E} = 109 \times Δ \left[at%\right] Cr + 64 \times Δ \left[at%\right] Cu + 259 \times Δ \left[at%\right] Mo + 45 \times Δ \left[at%\right] Ni ,$ wobei Δ[at%]X der den Schwellwert übersteigende Gehalt des Einflusselements X ist und wobei die für das jeweilige Einflusselement X ermittelte Steigung, welche dessen Einfluss auf die Zugfestigkeit Rₘ abbildet, um ±25% von dem angegebenen Wert abweichen kann.

Eine vorteilhafte Weiterbildung sieht vor, dass im Korrekturmodell der Kompensationsfaktor F_{K} in MPa für die Kompensationselemente unter Verwendung der folgenden Formel berechnet wird: FK = 207 × Δ[at%]C + 25 × Δ[at%]Si + 96 × Δ[at%]Mn + (3913 - 34544 × [at%]NbZiel) × Δ[at%]Nb2 + 1204 × Δ[at%]Ti - 277 × Δ[at%]N, wobei [at%]Y_{Ziel} der Zielwert des Kompensationselements Y und Δ[at%]Y die Abweichung des im Korrekturmodell zum Ausgleich der Zugfestigkeit Rₘ berechneten Kompensationselementgehalts vom Zielwert des Kompensationselements Y ist und wobei die für das jeweilige Kompensationselement Y ermittelte Steigung, welche dessen Einfluss auf die Zugfestigkeit Rₘ abbildet, um ±25% von dem angegebenen Wert abweichen kann.

Eine vorteilhafte Weiterbildung sieht vor, dass die Summe aus dem Einflussfaktor und dem Kompensationsfaktor Δ*F* = |*F_{E} + F_{K}*| minimiert wird, sodass ΔF einen Maximalwert, insbesondere 20 MPa, bevorzugt 10 MPa, besonders bevorzugt 5 MPa nicht überschreitet.

Eine vorteilhafte Weiterbildung sieht vor, dass die Kompensationselementgehalte um maximal 30%, bevorzugt 25%, besonders bevorzugt 20% gegenüber dem Zielwert abgesenkt werden.

Eine vorteilhafte Weiterbildung sieht vor, dass eine Auswahl der Kompensationselemente nach einem, mehreren oder allen der nachfolgenden Kriterien erfolgt: Möglichkeit der Elementkorrektur innerhalb des Zielbereichs, Treffsicherheit des jeweiligen Kompensationselements, Zeitpunkt der Legierungseinstellung, Stärke der Abhängigkeit der Zugfestigkeit Rₘ von dem jeweiligen Kompensationselement, Differenz aus dem zum Ausgleich der Zugfestigkeit Rₘ berechneten Kompensationselementgehalt und dem Zielwert des Kompensationselements, Wahl weiterer Kompensationselemente, Kosten des jeweiligen Kompensationselements.

Eine vorteilhafte Weiterbildung sieht vor, dass eine Auswahl der Kompensationselemente nach nachfolgenden Kriterien erfolgt: Möglichkeit der Elementkorrektur innerhalb des Zielbereichs, Treffsicherheit des jeweiligen Kompensationselements, Zeitpunkt der Legierungseinstellung, Stärke der Abhängigkeit der Zugfestigkeit Rₘ von dem jeweiligen Kompensationselement, Differenz aus dem zum Ausgleich der Zugfestigkeit Rₘ berechneten Kompensationselementgehalt und dem Zielwert des Kompensationselements, Wahl weiterer Kompensationselemente und/oder Kosten des jeweiligen Kompensationselements.

Eine vorteilhafte Weiterbildung sieht vor, dass Einflusselemente Elemente sind, welche im Einsatzstoff Spurenelemente oder Begleitelemente oder Legierungselemente sind und deren Gehalt einen definierten Schwellwert überschreitet, und Kompensationselemente Elemente sind, die reguläre Legierungselemente der Ziellegierung sind oder andere Elemente sind.

Eine vorteilhafte Weiterbildung sieht vor, dass die Probenentnahme und Analyse, insbesondere ab einem Abstich von Rohstahl zumindest in der Schmelze des letzten primärmetallurgischen Aggregates der Erzeugungsroute und nach jeder weiteren Zugabe von Elementen durchgeführt werden.

Eine vorteilhafte Weiterbildung sieht vor, dass die kaltgewalzten, niedrig- oder mikrolegierten Stähle eine Dicke von 0,30 bis 3,0 mm und eine Zugfestigkeit Rₘ von 310 bis 780 MPa aufweisen.

Eine vorteilhafte Weiterbildung sieht vor, dass die Ziellegierung die folgende Ziellegierungszusammensetzung in at.-% umfasst:

| | |
|---|---|
| C | 0,09-0,60 |
| Si | ≤ 1,20 |
| Mn | 0,20-1,80 |
| P | ≤ 0,055 |
| S | ≤ 0,045 |
| Al | 0,03-0,40 |
| Ti | ≤ 0,18 |
| Nb | ≤ 0,06 |
| Ti+Nb | 0,003-0,14 |
| Cu | ≤ 0,17 |
| Cr | ≤ 0,26 |
| Mo | ≤ 0,05 |
| Ni | ≤ 0,28 |
| N | ≤ 0,08 |

Rest Eisen und erschmelzungsbedingte Verunreinigungen.

Eine vorteilhafte Weiterbildung sieht vor, dass die Schwellwerte der Einflusselemente in at% folgende sind:

| | |
|---|---|
| Cu | 0,02; |
| Cr | 0,04; |
| Mo | 0,01; |
| Ni | 0,03, |

wobei, wenn zumindest ein Analysen-Ist-Wert zumindest eines Einflusselements den jeweiligen Schwellwert übersteigt, eine Kompensation der Wirkung des zumindest einen Einflusselements auf die Zugfestigkeit Rₘ durch angepasste Kompensationselementgehalte notwendig wird.

Die Erfindung betrifft ferner ein Steuersystem zur Herstellung von kaltgewalzten, niedrig- oder mikrolegierten Stählen eines gewünschten Bereichs der Zugfestigkeit Rₘ und zur Durchführung des erfindungsgemäßen Verfahrens, wobei das Steuersystem zwei Komponenten umfasst: ein Prognosemodell und ein Korrekturmodell, wobei das Prognosemodell dazu ausgelegt ist, einen Einfluss von Einflusselementen auf die Zugfestigkeit Rₘ zu berechnen, und das Korrekturmodell dazu ausgelegt ist, zum Ausgleich der Zugfestigkeit Rₘ erforderliche Kompensationselementgehalte sowie einen Einfluss der Kompensationselemente auf die Zugfestigkeit Rₘ zu berechnen.

Die Erfindung wird beispielhaft anhand einer Zeichnung dargestellt. Es zeigen dabei:
- Figur 1: einen Graphen, welcher die Begrifflichkeiten Schwellwert, Analysen-Ist-Wert, Abweichung des Analysen-Ist-Werts vom Schwellwert des Einflusselements X, Δ[at%]X, und Maximalgehalt bezüglich der Einflusselemente veranschaulicht;
- Figur 2: einen Graphen, welcher die Begrifflichkeiten Zielwert, adaptierter Zielwert, Abweichung des im Korrekturmodell zum Ausgleich der Zugfestigkeit Rₘ berechneten Kompensationselementgehalts (= adaptierter Zielwert) vom Zielwert des Kompensationselements Y, Δ[at%]Y, und Maximalgehalt bezüglich der Kompensationselemente veranschaulicht;
- Figur 3: Maximalgehalte und Analysen-Ist-Werte der Einflusselemente vor und nach dem dynamischen Legieren in zwei Schritten;
- Figur 4: Maximalgehalte und Analysen-Ist-Werte der Kompensationselemente vor und nach dem dynamischen Legieren in zwei Schritten.

Im Zuge einer beispielhaften Ausführungsform dynamischen Legierens nach der Erfindung werden folgende Schritte durchgeführt:
1) Erfassen der Analysen-Ist-Werte und Vergleich mit Schwellwerten. Abhängig hiervon ist die Entscheidung zu treffen, ob dynamisches Legieren notwendig ist. Wenn dies der Fall ist, wird mit Schritt 2 fortgefahren;
2) Berechnung des Einflussfaktors F_{E} im Prognosemodell (Maß für den Einfluss der Einflusselemente auf die Zugfestigkeit Rₘ);
3) Berechnung adaptierter Kompensationselementgehalte im Korrekturmodell (adaptierte Zielwerte), wobei diese um maximal 30%, bevorzugt 25%, besonders bevorzugt 20% gegenüber ihren eigentlichen Zielwerten abgesenkt werden dürfen;
4) Berechnung des Kompensationsfaktor F_{K} im Korrekturmodell (Maß für den Einfluss der Kompensationselemente auf die Zugfestigkeit Rₘ);
5) Bildung der Summe ΔF aus dem Einflussfaktor F_{E} und dem Kompensationsfaktor F_{K};
6) Wiederholung der Schritte 3) bis 5), bis ΔF so weit minimiert ist, dass es einen Maximalwert, insbesondere 20 MPa, bevorzugt 10 MPa, besonders bevorzugt 5 MPa nicht überschreitet;
7) Zulegieren der Kompensationselementgehalte auf die adaptierten Zielwerte, um die Zugfestigkeit Rₘ des Endprodukts in einen Zielbereich zu bringen.

Nach dem Erfassen der Analysen-Ist-Werte wird, wenn die Einflusselementgehalte ihre jeweiligen Schwellwerte übersteigen, zunächst mit dem Prognosemodell der Einflussfaktor F_{E} in MPa für die Einflusselemente unter Verwendung der folgenden Formel berechnet: ${F}_{E} = 109 \times Δ \left[at%\right] Cr + 64 \times Δ \left[at%\right] Cu + 259 \times Δ \left[at%\right] Mo + 45 \times Δ \left[at%\right] Ni ,$ wobei Δ[at%]X der den Schwellwert übersteigende Gehalt des Einflusselements X ist und wobei die für das jeweilige Einflusselement X ermittelte Steigung, welche dessen Einfluss auf die Zugfestigkeit Rₘ abbildet, um ±25% von dem angegebenen Wert abweichen kann.

Hierbei sind Chrom, Kupfer, Molybdän und Nickel Einflusselemente.

Anschließend werden mithilfe des Korrekturmodells adaptierte Kompensationselementgehalte (adaptierte Zielwerte) bestimmt, wobei diese um maximal 30%, bevorzugt 25%, besonders bevorzugt 20% gegenüber ihren eigentlichen Zielwerten abgesenkt werden dürfen, und der Kompensationsfaktor F_{K} in MPa für die Kompensationselemente unter Verwendung der folgenden Formel berechnet: FK = 207 × Δ[at%]C + 25 × Δ[at%]Si + 96 × Δ[at%]Mn + (3913 - 34544 × [at%]NbZiel) × Δ[at%]Nb2 + 1204 × Δ[at%]Ti - 277 × Δ[at%]N, wobei [at%]Y_{Ziel} der Zielwert des Kompensationselements Y und Δ[at%]Y die Abweichung des im Korrekturmodell zum Ausgleich der Zugfestigkeit Rₘ berechneten Kompensationselementgehalts vom Zielwert des Kompensationselements Y ist und wobei die für das jeweilige Kompensationselement Y ermittelte Steigung, welche dessen Einfluss auf die Zugfestigkeit Rₘ abbildet, um ±25% von dem angegebenen Wert abweichen kann.

Hierbei sind Kohlenstoff, Silizium, Mangan, Niob, Titan und Stickstoff Kompensationselemente.

Die Summe aus dem Einflussfaktor F_{E} und dem Kompensationsfaktor F_{K} wird folgendermaßen bestimmt: $Δ F = \left|{F}_{E}+{F}_{K}\right| .$ ΔF soll dabei minimiert werden, sodass ΔF einen Maximalwert, insbesondere 20 MPa, bevorzugt 10 MPa, besonders bevorzugt 5 MPa nicht überschreitet.

Die Berechnung adaptierter Kompensationselementgehalte (adaptierter Zielwerte) wird im Korrekturmodell iteriert, bis eine Lösung gefunden wird, welche obige Bedingung für ΔF erfüllt.

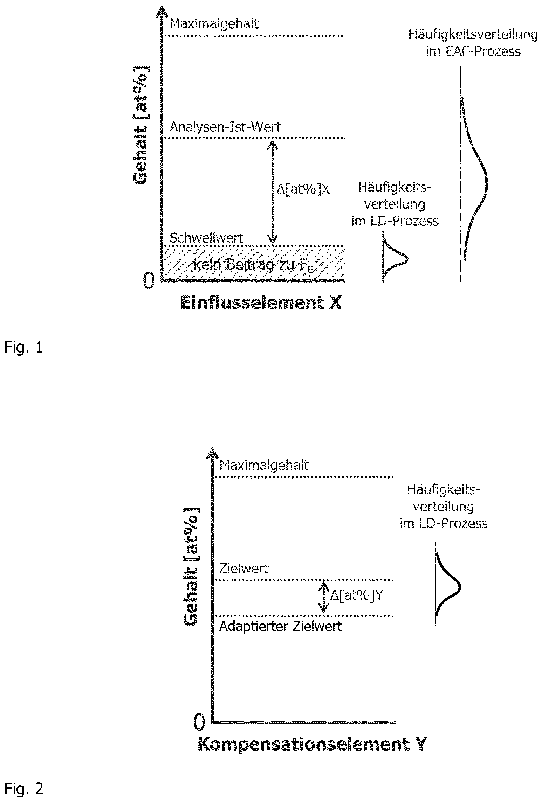

Die Figuren 1 und 2 veranschaulichen die Begrifflichkeiten Schwellwert, Analysen-Ist-Wert, Abweichung des Analysen-Ist-Werts vom Schwellwert des Einflusselements X, Δ[at%]X, und Maximalgehalt bezüglich der Einflusselemente (Figur 1) und Zielwert, adaptierter Zielwert, Abweichung des im Korrekturmodell zum Ausgleich der Zugfestigkeit Rₘ berechneten Kompensationselementgehalts (= adaptierter Zielwert) vom Zielwert des Kompensationselements Y, Δ[at%]Y, und Maximalgehalt bezüglich der Kompensationselemente (Figur 2).

Δ[at%]X ergibt sich aus der Differenz aus Analysen-Ist-Wert und Schwellwert, d.h. Δ[at%]X = Analysen-Ist-Wert - Schwellwert. Dementsprechend ergibt sich für Δ[at%]X bei Überschreitung des Schwellwerts immer ein positives Vorzeichen. Δ[at%]Y ergibt sich dagegen aus der Differenz aus adaptierten Zielwert und eigentlichem Zielwert (ohne dynamisches Legieren), d.h. Δ[at%]Y = adaptierter Zielwert - Zielwert. Dementsprechend ergibt sich für Δ[at%]Y i.d.R. ein negatives Vorzeichen.

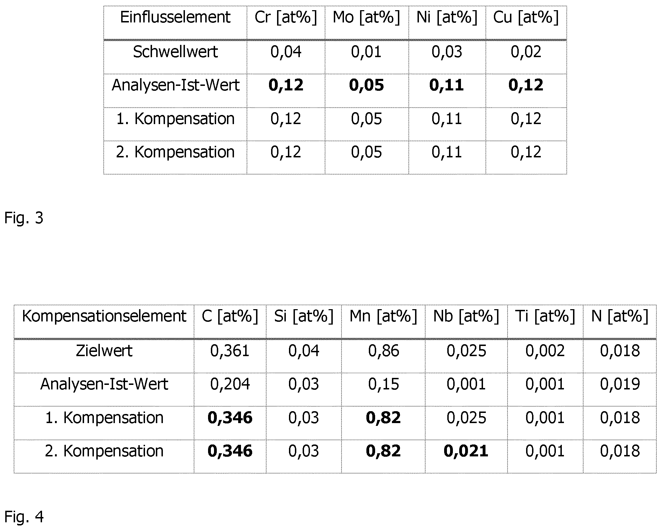

In den Figuren 3 und 4 sind Einfluss- und Kompensationselementgehalte für eine beispielhafte Ausführungsform des erfindungsgemäßen Verfahrens gezeigt.

Figur 3 zeigt eine Tabelle mit den Schwellwerten und den Analysen-Ist-Werten bzw. Analysen-Ist-Gehalten der Einflusselemente Chrom, Molybdän, Nickel und Kupfer.

Der Schwellwert ist jener Gehalt, ab dem ein Eingriff durch dynamisches Legieren notwendig wird, d.h. überschreitet der Analysen-Ist-Wert eines Einflusselements diesen Wert muss sein Beitrag zum Einflussfaktor FE berücksichtigt werden und sein Einfluss durch eine Adaption der Kompensationselementgehalte kompensiert werden. Dieser Gehalt, ab dem eingegriffen wird, ist üblicherweise deutlich niedriger als der durch die Ziellegierung vorgegeben Maximalgehalt, bis zu dem ein Eingriff erfolgen kann.

Schwellwerte der Einflusselemente in at% sind:

| | |
|---|---|
| Cu | 0,02; |
| Cr | 0,04; |
| Mo | 0,01; |
| Ni | 0,03. |

Übersteigen die Analysen-Ist-Analyse-Werte bzw. Analysen-Ist-Gehalte ihr jeweiligen Schwellwerte, muss dies durch eine Anpassung der Kompensationselementgehalte kompensiert werden, d.h. es muss dynamisches Legieren stattfinden.

Im vorliegenden Beispiel (Figur 3) sind die Analysen-Ist-Werte bzw. Analysen-Ist-Gehalte der Einflusselemente gegenüber ihren Schwellwerten deutlich erhöht. Dementsprechend ist ein Eingriff in Form des dynamischen Legierens notwendig, d.h. die Gehalte bzw. Zielwerte der Kompensationselemente müssen im Korrekturmodell entsprechend adaptiert werden, um die Zugfestigkeit Rₘ in den Zielbereich zu bringen.

Für den Einflussfaktor F_{E} ergibt sich hierbei ein Wert von 29 MPa.

Figur 4 zeigt eine Tabelle mit den Zielwerten, den Analysen-Ist-Werten und den im Rahmen der Kompensation teilweise angepassten Gehalten bzw. Zielwerten der Kompensationselemente Kohlenstoff, Silizium, Mangan, Niob, Titan und Stickstoff (hier Kompensation in zwei Schritten).

Im ersten Kompensationsschritt werden die Gehalte von Kohlenstoff und Mangan im Korrekturmodell neu berechnet (adaptierte Zielwerte). Dabei ergibt sich ein Kompensationsfaktor F_{K} von -8 MPa. Entsprechend beträgt die Summe aus dem Einflussfaktor F_{E} und dem Kompensationsfaktor F_{K} (ΔF) 21 MPa. Dieser Wert ist zu hoch, d.h. die berechnete Kompensation ist nicht geeignet bzw. reicht nicht aus. Daher ist ein weiterer Kompensationsschritt durchzuführen.

Im zweiten Kompensationsschritt wird zusätzlich der Gehalt von Niob angepasst. Nun ergibt sich ein Kompensationsfaktor F_{K} von -30 MPa.

Entsprechend beträgt die Summe aus dem Einflussfaktor F_{E} und dem Kompensationsfaktor F_{K} (ΔF) 1 MPa. Damit ist ΔF weitgehend minimiert und überschreitet nicht den Maximalwert von insbesondere 20 MPa, bevorzugt 10 MPa, besonders bevorzugt 5 MPa. Somit ist die im zweiten Kompensationsschritt berechnete Korrektur geeignet, um die Zugfestigkeit Rₘ in einen Zielbereich zu bringen. Das Zulegieren erfolgt entsprechend der im Korrekturmodell berechneten adaptierten Gehalte an bzw. Zielwerte für Kohlenstoff, Mangan und Niob.

Die Steigungen (d.h. Einflüsse der Elemente auf die Zugfestigkeit Rₘ) ergeben sich aus praktischen Versuchen, beispielsweise Laborversuchen, sowie aus der statistischen Auswertung von Prozess- und Versuchsdaten der Serienproduktion.

Bei einem Laborversuch wird in einer Versuchsanlage, die alle wesentlichen Komponenten eines Walzwerkes zur Herstellung von Flachprodukten umfasst, ein kleintechnisch hergestellter Gussblock erwärmt und dann unter Vorgabe einer festgelegten Stichabfolge, definiert durch die einzelnen Stichabnahmen sowie einer oder mehrerer Zieltemperaturen, die während bzw. am Ende des Walzprozesses eingehalten werden sollen, auf die gewünschte Warmbanddicke gewalzt. Der so gewonnene Walzstreifen wird anschließend in Wasser oder an Luft abgekühlt. Der warmgewalzte abgekühlte Walzstreifen wird im Anschluss kaltgewalzt und daraus Proben in einem Glühsimulator wärmebehandelt. Aus den geglühten Proben werden eine oder mehrere Zugproben entnommen, deren mechanische Eigenschaften wie Streckgrenze, Zugfestigkeit und Bruchdehnung auf konventionellen Zugprüfmaschinen bestimmt werden können.

Die im Versuch gewonnenen Daten dienen als Basis für die Entwicklung eines Modells zur Prognose der Zugfestigkeit Rₘ in Abhängigkeit der Legierungselemente, der Begleit- und Spurenelemente sowie der Herstellroute der Bleche. Das Prognosemodell beruht somit auf werkstoffkundlich und insbesondere physikalisch und metallkundlich abgesicherten Grundlagen.

Zur Berechnung der Steigung eines bestimmten Elements können beispielsweise Versuche an zwei Proben, die sich nur in einem Elementgehalt unterscheiden, durchgeführt werden. Aus dem sich ergebenden Delta wird dann die Steigung für dieses eine Element berechnet.

Die kleintechnische Herstellung der Gussblöcke erfolgt in einem eigens dafür konzipierten Schmelzofen. Dabei können die Legierungselemente über das im großtechnischen Herstellprozess angewendete Legierungsfenster hinaus variiert werden. Auch bei der Vorgabe der Prozessparameter (wie z.B. Erwärmungstemperaturen, Walztemperaturen und Kühlraten) können Werte realisiert werden, die im großtechnischen Prozess nicht üblich sind, oder an den Produktionsanlagen technisch gar nicht realisierbar sind.

Mit dieser Vorgehensweise, dass nämlich sowohl Legierungselementgehalte als auch Prozessparameter zur Anwendung gebracht werden, die über die üblichen Grenzen der produzierten Bleche hinausgehen, erhält man gut abgesicherte Modellparameter für das Prognosemodell und/oder Korrekturmodell. Für das dynamische Legieren ist der Einfluss der Legierungs- und Begleitelemente essenziell.

Nachdem bei einem Großteil der Bleche nach erfolgter Produktion eine Zugprobe entnommen wird, können diese Werte zur Verifikation des Prognosemodells, aber auch zur Feinjustierung der Parameter herangezogen werden.

Diese Feinjustierung der Parameter, genaugenommen der Steigungswerte, erfolgt mit computerunterstützen Methoden, indem ausgewählte Parameter der oben beschriebenen Modellgleichungen mit Hilfe der Methode der kleinsten Quadrate ermittelt werden. Auch der Einsatz von Methoden der künstlichen Intelligenz (z.B. neuronale Netze) ist für diese Feinjustierung denkbar. Da diese Feinjustierung immer auf Basis von gemessenen Daten erfolgt, kann sich eine Variation der Steigungswerte im Bereich ±25% ergeben. Durch die Messfehler der Prozessdaten aber auch der gemessenen mechanischen Eigenschaften selbst, ist eine entsprechende Ungenauigkeit und damit Streuung der Steigungswerte nicht vermeidbar. Die Streuung selbst wird umso geringer, je größer die Datensätze sind, die zur Feinjustierung herangezogen werden können.

Somit können mit dem vorliegenden Verfahren, welches ein dynamisches, variables und flexibles Legieren ermöglicht, konstante Produkteigenschaften auch bei größeren Schwankungen der Einflusselementfracht gewährleistet werden.

## Patentansprüche

1. Verfahren zur Herstellung von kaltgewalzten, niedrig- oder mikrolegierten Stählen eines Zielbereichs der Zugfestigkeit Rₘ, welche durch eine Ziellegierung umfassend Eisen, sowie Begleitelemente und Legierungselemente eingestellt werden, wobei ein Lichtbogenofen und/oder LD-Konverter unter Zugabe von zumindest Stahlschrott beladen wird und der Inhalt des Lichtbogenofens und/oder des LD-Konverters erschmolzen wird, wobei aus einer Schmelze eines letzten primärmetallurgischen Aggregates einer Erzeugungsroute mindestens eine Probe entnommen und durch deren Analyse Gehalte der Begleit- und Legierungselemente bestimmt werden, wobei Analysen-Ist-Werte der Begleit- und Legierungselementgehalte an ein Steuersystem geleitet werden, wobei Begleitelemente Elemente sind, welche in die Schmelze durch Einsatzstoffe eingebracht werden und deren Gehalte einen durch die Ziellegierung definierten Maximalgehalt nicht überschreiten dürfen und Legierungselemente Elemente sind, die gezielt entsprechend durch die Ziellegierung definierter Zielwerte zugesetzt werden,
**dadurch gekennzeichnet, dass**
zwischen Einfluss- und Kompensationselementen unterschieden wird, wobei
Einflusselemente und Kompensationselemente Elemente sind, welche einen Einfluss bzw. eine Wirkung auf die Zugfestigkeit Rₘ haben, wobei
der Unterschied der Analysen-Ist-Werte von Schwellwerten bezüglich der Einflusselemente erfasst wird, wobei
die Größe des Einflusses der Abweichung der Analysen-Ist-Werte von den Schwellwerten aller Einflusselemente auf die Zugfestigkeit Rₘ erfasst wird, wobei
zumindest ein Kompensationselement ermittelt wird, welches angepasst, d.h. gemäß einem korrigierten Zielwert, derart in die Schmelze zulegiert wird, dass die Zugfestigkeit Rₘ in einen Zielbereich gebracht und der Einfluss des Einflusselements hierdurch kompensiert wird,
wobei die Einflusselemente, deren Einfluss auf die Zugfestigkeit Rₘ kompensiert werden soll, eines, mehrere oder alle aus der Gruppe von Cr, Cu, Mo, Ni umfassen und wobei als Kompensationselemente eines, mehrere oder alle aus der Gruppe von C, Si, Mn, Nb, Ti, N gewählt werden.

2. Verfahren nach Anspruch 1, **dadurch gekennzeichnet, dass** im Steuersystem zwei Komponenten verwendet werden: ein Prognosemodell und ein Korrekturmodell, wobei mit dem Prognosemodell ein Einflussfaktor F_{E} ermittelt wird, welcher die Größe des Einflusses der Abweichung der Analysen-Ist-Werte von den Schwellwerten aller Einflusselemente auf die Zugfestigkeit Rₘ beschreibt, und mit dem Korrekturmodell zum Ausgleich der Zugfestigkeit Rₘ erforderliche Kompensationselementgehalte berechnet werden sowie ein Kompensationsfaktor F_{K} ermittelt wird, welcher die Größe eines Einflusses einer Abweichung der berechneten Kompensationselementgehalte von den Zielwerten aller Kompensationselemente auf die Zugfestigkeit Rₘ beschreibt.

3. Verfahren nach Anspruch 1 oder 2, **dadurch gekennzeichnet, dass** zur Berechnung der Zugfestigkeit Rₘ in MPa die folgende Formel verwendet wird: Rm = 235 + 207 × [at%]C + 25 × [at%]Si + 96 × [at%]Mn + 243 × [at%]P - 104 × [at%]S + 18 × [at%]Al - 277 × [at%]N + 3913 × [at%]Nb - 17272 × [at%]Nb2 + 1204 × [at%]Ti + 109 × [at%]Cr + 64 × [at%]Cu + 259 × [at%]Mo + 45 × [at%]Ni, wobei [at%]i der Gehalt des jeweiligen Elements i ist und wobei die für das jeweilige Element i ermittelte Steigung, welche dessen Einfluss auf die Zugfestigkeit Rₘ abbildet, um ±25% von dem angegebenen Wert abweichen kann.

4. Verfahren nach einem der vorhergehenden Ansprüche, **dadurch gekennzeichnet, dass** im Prognosemodell der Einflussfaktor F_{E} in MPa für die Einflusselemente unter Verwendung der folgenden Formel berechnet wird: ${F}_{E} = 109 \times Δ \left[at%\right] Cr + 64 \times Δ \left[at%\right] Cu + 259 \times Δ \left[at%\right] Mo + 45 \times Δ \left[at%\right] Ni ,$ wobei Δ[at%]X der den Schwellwert übersteigende Gehalt des Einflusselements X ist und wobei die für das jeweilige Einflusselement X ermittelte Steigung, welche dessen Einfluss auf die Zugfestigkeit Rₘ abbildet, um ±25% von dem angegebenen Wert abweichen kann.

5. Verfahren nach einem der vorhergehenden Ansprüche, **dadurch gekennzeichnet, dass** im Korrekturmodell der Kompensationsfaktor F_{K} in MPa für die Kompensationselemente unter Verwendung der folgenden Formel berechnet wird:

6. FK = 207 × Δ[at%]C + 25 × Δ[at%]Si + 96 × Δ[at%]Mn + (3913 - 34544 × [at%]NbZiel) × Δ[at%]Nb2 + 1204 × Δ[at%]Ti - 277 × Δ[at%]N, wobei [at%]Y_{Ziel} der Zielwert des Kompensationselements Y und Δ[at%]Y die Abweichung des im Korrekturmodell zum Ausgleich der Zugfestigkeit Rₘ berechneten Kompensationselementgehalts vom Zielwert des Kompensationselements Y ist und wobei die für das jeweilige Kompensationselement Y ermittelte Steigung, welche dessen Einfluss auf die Zugfestigkeit Rₘ abbildet, um ±25% von dem angegebenen Wert abweichen kann.

7. Verfahren nach einem der vorhergehenden Ansprüche, **dadurch gekennzeichnet, dass** die Summe aus dem Einflussfaktor und dem Kompensationsfaktor Δ*F* = |*F_{E}* + *F_{K}*| minimiert wird, sodass ΔF einen Maximalwert, insbesondere 20 MPa, bevorzugt 10 MPa, besonders bevorzugt 5 MPa nicht überschreitet.

8. Verfahren nach einem der vorhergehenden Ansprüche, **dadurch gekennzeichnet, dass** die Kompensationselementgehalte um maximal 30%, bevorzugt 25%, besonders bevorzugt 20% gegenüber dem Zielwert abgesenkt werden.

9. Verfahren nach einem der vorhergehenden Ansprüche, **dadurch gekennzeichnet, dass** eine Auswahl der Kompensationselemente nach einem, mehreren oder allen der nachfolgenden Kriterien erfolgt: Möglichkeit der Elementkorrektur innerhalb des Zielbereichs, Treffsicherheit des jeweiligen Kompensationselements, Zeitpunkt der Legierungseinstellung, Stärke der Abhängigkeit der Zugfestigkeit Rₘ von dem jeweiligen Kompensationselement, Differenz aus dem zum Ausgleich der Zugfestigkeit Rₘ berechneten Kompensationselementgehalt und dem Zielwert des Kompensationselements, Wahl weiterer Kompensationselemente, Kosten des jeweiligen Kompensationselements.

10. Verfahren nach einem der vorhergehenden Ansprüche, **dadurch gekennzeichnet, dass** Einflusselemente Elemente sind, welche im Einsatzstoff Spurenelemente oder Begleitelemente oder Legierungselemente sind und deren Gehalt einen definierten Schwellwert überschreitet, und Kompensationselemente Elemente sind, die reguläre Legierungselemente der Ziellegierung sind oder andere Elemente sind.

11. Verfahren nach einem der vorhergehenden Ansprüche, **dadurch gekennzeichnet, dass** die Probenentnahme und Analyse, insbesondere ab einem Abstich von Rohstahl zumindest in der Schmelze des letzten primärmetallurgischen Aggregates der Erzeugungsroute und nach jeder weiteren Zugabe von Elementen durchgeführt werden.

12. Verfahren nach einem der vorhergehenden Ansprüche, **dadurch gekennzeichnet, dass** die kaltgewalzten, niedrig- oder mikrolegierten Stähle eine Dicke von 0,30 bis 3,0 mm und eine Zugfestigkeit Rₘ von 310 bis 780 MPa aufweisen.

13. Verfahren nach einem der vorhergehenden Ansprüche, **dadurch gekennzeichnet, dass** die Ziellegierung die folgende Ziellegierungszusammensetzung in at.-% umfasst:
| | |
|---|---|
| C | 0,09-0,60 |
| Si | ≤ 1,20 |
| Mn | 0,20-1,80 |
| P | ≤ 0,055 |
| S | ≤ 0,045 |
| Al | 0,03-0,40 |
| Ti | ≤ 0,18 |
| Nb | ≤ 0,06 |
| Ti+Nb | 0,003-0,14 |
| Cu | ≤ 0,17 |
| Cr | ≤ 0,26 |
| Mo | ≤ 0,05 |
| Ni | ≤ 0,28 |
| N | ≤ 0,08 |
Rest Eisen und erschmelzungsbedingte Verunreinigungen.

14. Verfahren nach einem der vorhergehenden Ansprüche, **dadurch gekennzeichnet, dass** die Schwellwerte der Einflusselemente in at% folgende sind:
| | |
|---|---|
| Cu | 0,02; |
| Cr | 0,04; |
| Mo | 0,01; |
| Ni | 0,03, |
wobei, wenn zumindest ein Analysen-Ist-Wert zumindest eines Einflusselements den jeweiligen Schwellwert übersteigt, eine Kompensation der Wirkung des zumindest einen Einflusselements auf die Zugfestigkeit Rₘ durch angepasste Kompensationselementgehalte notwendig wird.

15. Steuersystem zur Herstellung von kaltgewalzten, niedrig- oder mikrolegierten Stählen eines gewünschten Bereichs der Zugfestigkeit Rₘ und zur Durchführung des Verfahrens nach einem der vorhergehenden Ansprüche, **dadurch gekennzeichnet, dass** das Steuersystem zwei Komponenten umfasst: ein Prognosemodell und ein Korrekturmodell, wobei das Prognosemodell dazu ausgelegt ist, einen Einfluss von Einflusselementen auf die Zugfestigkeit Rₘ zu berechnen, und das Korrekturmodell dazu ausgelegt ist, zum Ausgleich der Zugfestigkeit Rₘ erforderliche Kompensationselementgehalte sowie einen Einfluss der Kompensationselemente auf die Zugfestigkeit Rₘ zu berechnen.
